# EUROPEAN PATENT SPECIFICATION

(11) **EP 0 316 444 B1**
(45) Date of publication and mention of the grant of the patent: **02.08.1995**
(21) Application number: 88906345.9
(22) Date of filing: 06.06.1988
(51) Int. Cl.: C07H 15/12, C12N 1/16, C12N 15/00, C12P 21/00, C12N 9/34

(54) **YEAST EXPRESSION VECTOR**
EXPRESSIONSVEKTOR FÜR HEFE
VECTEUR D'EXPRESSION DE LEVURE

(30) Priority: 06.06.1987 US 58818
(43) Date of publication of application: 24.05.1989
(73) Proprietor: OMNIGENE INC, Cambridge Massachusetts 02139-9002 (US)
(72) Inventor: DAVES, Robert, S., Syracuse, NY 13203 (US); YOCUM, Robert, Rogers, Arlington, MA 02174 (US)
(74) Representative: Deans, Michael John Percy
(86) International application number: US8801952
(87) International publication number: WO8809795

(56) References cited:
- EP-A- 0 103 409
- WO-A-86/07091
- NUNBERG, JACK H. et al. WO 84/02921 published 2 August, 1984 (see especially pages 11 and 14, and Fig. 7)
- COHEN, REGINA et al: "Identification of a regulatory region that mediates Glucose-dependent induction of the Saccharomyces cerevisiae enolase gene ENO 2"; pages 2287-2297; published July 1986 by American Society of Microbiology (Washington, D.C.)
- HOLLAND, MICHAEL J. et al; "The Primary Structure of Two Yeast Enolase Genes:J. Biol. Chem. Vol. 256, No. 3 pages 1385-1395; published February 10, 1981 by the American Society of Biological Chemists (Baltimore, MD) see entire document especially Figs. 5 and 7
- UEMURA, HIROSHI et al: "Nucleotide sequence of the 5' flanking region responsible for the Enhancement of the expression of Yeast Enolase 1 gene"; J. Biochem. Vol. 98 No. 3 pages 859-862; published September 1985 by The Japanese Biochemial Society (Tokoyo, Japan) see entire document especially Fig. 4
- INNIS, M. A. et al: "Expression, glycosylation, and secretion of an Aspergillus glucoamylase by Saccharomyces cerevisiae"; Science Vol. 228 No. 4695 pages 21-26, published 5 April 1985 by The American Association for the Advancement of Science (Washington, D.C.) see entire document, especially page 21 and Fig. 1
- GUARENTE, LEONARD et al: "A GAL10-CYC1 hybrid yeast promotor identifies the GAL4 regulatory region as an upstream site"; Proc.Natl. Acad. Sci. Vol. 79 No. 23 pages 7410-7414 published December 1982 by the National Academy of Science (Washington, D.C.) see entire document, especially Fig. 2
- Proc. Natl. Acad. Sci. USA, 1986 Vol. 82, pp. 8557-8561
- The EMBO Journal, 1985 Vol. 4 No. 12, pp. 3273-3280
- Molecular and Cellular Biology, 1989, Vol. 9, No. 12, pp. 5526-5524

## Description

This invention relates to genetic engineering of yeast, and to the use of certain promoters to improve the results of such genetic engineering.

One of the goals of genetic engineering in yeast is to construct strains of yeast that produce large amounts of a desired protein or enzyme. A particular protein or enzyme may be of interest as a product itself (for example, human alpha interferon), or a protein or enzyme may be of interest because it facilitates production by the host yeast of a desired metabolite (for example, ethanol or an amino acid) or production of a food or beverage product (for example, beer, wine, bread, or spirits). Although it is now routine to obtain some level of expression in yeast of any desired gene into its corresponding protein, the attainment of levels of expression high enough to allow a process to be performed profitably is often difficult.

The term "promoter", as used in connection with the expression of genes in yeast, has a meaning broader than the meaning of the term when used in connection with E. coli, the organism for which promoters were first described. The term "promoter", as used herein, means any DNA sequence which, when associated with a structural gene in a host yeast cell, increases, for that structural gene, one or more of 1) transcription, 2) translation, or 3) mRNA stability, compared to transcription, translation, or mRNA stability in the absence of the promoter sequence, under appropriate growth conditions (e.g., if the promoter is inducible, in the presence of the inducing substance). "mRNA stability" means longer half-life of mRNA.

Several strong promoters are known in the art and have been shown to be useful for expression of heterologous genes in yeast. (A "heterologous gene" means any gene that is not naturally functionally associated with a given promoter, whether that gene is derived from yeast or not.) For example, promoters naturally associated with the Saccharomyces cerevisiae genes TPI1 (triose phosphate isomerase), PGK1 (phosphoglycerate kinase), PYK1 (pyruvate kinase), TDH1, TDH2, and TDH3 (glyceraldehyde phosphate dehydrogenase or triose phosphate dehydrogenase), and EN01 (enolase 1) have been described as useful for expression of heterologous genes in yeast (Kawasaki, U.S. Patent No. 4,599,311; Kingsman and Kingsman, U.S. Patent No. 4,615,974; Burke et al.,. EPO Patent Application No. 84300091.0; and Nunberg et al., WPO Patent Application No. 84/02921). All of the above genes encode enzymes which are involved in the glycolytic pathway of yeast, and which are among the most abundant enzymes in the yeast cytoplasm (Brousse et al. (1985) Applied and Environmental Microbiology 50: 951).

Some yeast strains have two different enzymes which act as enolases in the glycolytic pathway. In S. cerevisiae, these two enzymes have been given the names enolase 1 and enolase 2, which are encoded by the ENO1 and ENO2 genes, respectively, each of which has associated with it its distinct promoter sequences (Holland et al. (1980) J. Biol. chem. 257: 7181 and Cohen et al. (1986) Mol. Cell Biol. 6: 2287). In S. cerevisiae, using glucose as a carbon source, enolase 2 is more abundant than enolase 1. As used herein, "enolase 2" refers to the most abundant enolase of any yeast strain using glucose as a carbon source, and "ENO2 promoter" refers to a promoter sequence naturally associated with the gene encoding the most abundant enolase in a yeast strain using glucose as a carbon source.

There have been some attempts to make hybrid yeast promoters. For example, Kingsman et al., EPO 258 067, describe work in which substitution of the PGK UAS with the GAL1,10 UAS confers galactose regulation on the PGK promoter, but insertion of the GAL1,10 UAS at a site upstream or downstream from the PGK UAS, so that both PGK and GAL1,10 UAS's were present in the promoter sequence, did not result in galactose regulation of PGK gene expression.

### Summary of the Invention

In general, the invention features a DNA sequence including a yeast EN02 promoter functionally fused to a heterologous gene, and a yeast cell transformed with that sequence. ("Functionally fused" means that transcription, translation, or mRNA stability of the heterologous gene is affected by the ENO2 promoter.) Generally, the DNA sequence of the invention is contained in a plasmid vector used to transform a host yeast cell, which is cultured to produce the protein or enzyme encoded by the heterologous gene. A preferred enzyme is a glucoamylase, which enables host yeast cells (preferably of the genus Saccharomyces) to utilize polysaccharides and thus renders the host yeast cells useful in the production of low carbohydrate "light" beer as described in Yocum et al. U.S. Serial No. 864,785, assigned to the same assignee as the present application, hereby incorporated by reference.

EN02 promoters can cause high heterologous gene expression levels in host yeast cells. In addition, EN02 promoter sequences of S. cerevisiae have the advantage of being regulatable: expression can be repressed 20-fold by growing the yeast on a nonfermentable carbon source, such as glycerol, lactate or ethanol (Cohen et al. (1986) Mol. Cell Biol. 6: 2287). Thus, in a case where the heterologous gene encodes a protein or enzyme inhibitory to yeast growth, expression of that gene can be repressed, to allow the cell mass to accumulate by growing the cells on a nonfermentable carbon source, and then expression of the desired gene induced by addition of glucose to the growth medium.

The invention also features a DNA sequence including a hybrid yeast promoter functionally fused to a heterologous gene, as well as a host yeast cell transformed with that sequence. The hybrid yeast promoter includes ENO2 promoter DNA functionally fused to a DNA sequence that contains a first UAS derived from a yeast promoter. Preferably, the ENO2 promoter DNA includes an ENO2 TATA region and an ENO2 UAS, and the first UAS may be derived from a yeast promoter other than the ENO2 yeast promoter or, alternatively, from an ENO2 promoter. The first UAS is located between the ENO2 UAS and the ENO2 TATA region. Most preferably, the first UAS derived from the non-ENO2 yeast promoter is a GAL1,10 UAS derived from the GAL1,10 DNA sequence of S. cerevisiae. In any given yeast strain, the GAL1,10 UAS is a DNA sequence located between the GAL1 yeast gene encoding galactokinase, and the GAL10 yeast gene, encoding UDP-galactose epimerase. The GAL1,10 UAS regulates both genes, which are divergently transcribed. GAL1,10 UAS's from other yeast species, particularly Saccharomyces yeasts such as S. carlsbergensis, can also be used. The DNA sequence of the S. carlsbergensis GAL1,10 UAS (Citron et al. (1984) J. Bact. 158: 269) is about 95% homologous with the DNA sequence of the GAL1,10 UAS of S. cerevisiae.

Hybrid yeast promoters of the invention are useful for achieving high heterologous gene expression in host yeast cells, and for allowing for the hybrid promoter to be regulated by a different carbon source; e.g., whereas the ENO2 promoter is normally induced by glucose, the hybrid ENO2-GAL1,10 promoter is repressed by glucose and induced by galactose.

Other features and advantages of the invention will be apparent from the following description of the preferred embodiments thereof, and from the claims.

### Description of the Preferred Embodiment

The drawings are first described.

### Drawings

Fig. 1 is a diagrammatic representation of a plasmid, pRD158, containing a glucoamylase gene under the transcriptional control of the prior art TPI1 promoter, and the derivation of the EN02 promoter-containing plasmid, pRD170, of the invention from pRD158.

Fig. 2 is a diagrammatic representation of the plasmids pRD219, a derivative of pRD170 containing the S. diastaticus DEX4 signal peptide encoding sequence; pSV30, a derivative of pRD219 containing the GAL1,10 upstream activating sequence inserted into the EN02 promoter; and pSV31, which is identical to pSV30 except that the GAL1,10 upstream activating sequence is inserted in the opposite orientation.

Fig. 3 is the DNA sequence of a BglII fragment, containing the GAL1,10 upstream activating sequence, which was inserted into pRD219 to form pSV30 and pSV31.

### Construction of pRD170

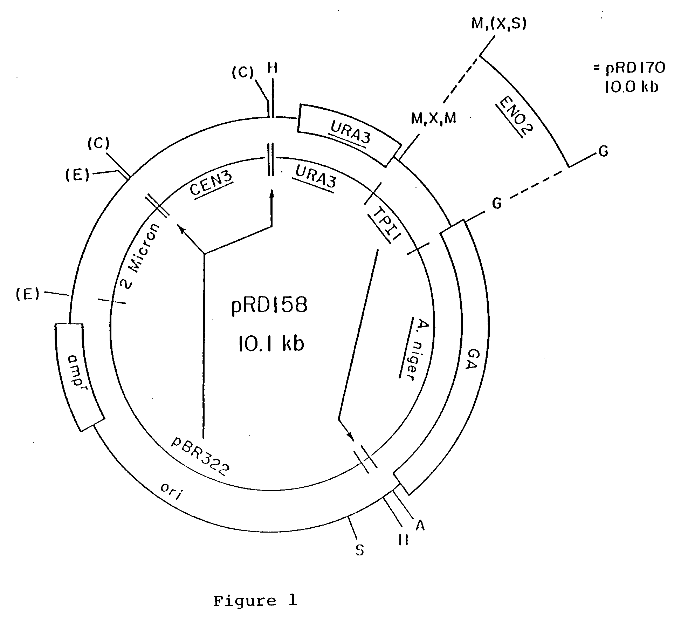

The first step in the construction of pRD170 (Fig. 1), in which the A. niger glucoamylase gene is under the transcriptional control of the EN02 promoter, was the isolation of the EN02 gene from Saccharomyces cerevisiae. Next, the EN02 promoter was isolated and substituted for the TPI1 promoter in pRD158. Finally, the resultant vector, pRD170, was used to transform host S. cerevisiae cells. In more detail, these steps were carried out as follows. (All manipulations of DNA, Escherichia coli and S. cerevisiae were accomplished by routine techniques well known in the art (Maniatis et al. (1982) Molecular Cloning Cold Spring Harbor Laboratory Press; and Sherman et al. (1981) Methods in Yeast Genetics, Cold Spring Harbor Laboratory Press). All DNA coordinates given herein are relative to the ATG start codon of the relevant coding sequence, with the A of the ATG start codon designated as +1.)

### Cloning of the EN02 Gene

Based on the published sequence of the S. cerevisiae EN02 gene, a single stranded oligonucleotide of 30 bases (+1 to +30 of the EN02 gene) was synthesized on an Applied Biosystems model 380A as recommended by the manufacturer. The sequence of the synthetic oligonucleotide is 5'-ATGGCTGTCTCTAAAGTTTACGCTAGATCC-3'. This 30-mer was labeled with ³²P at its 5' end and used as a hybridization probe to detect plasmids that contain the EN02 gene from a genomic S. cerevsiae gene bank made in the vector YEP24 (Carlson and Botstein (1982) Cell 28: 145). The colony hybridization method was used to detect replica E. coli colonies containing the desired plasmid. The presence of the EN02 gene and promoter on plasmid isolates was confirmed by demonstrating the presence of DNA restriction fragments predicted from the published DNA sequence, and by demonstrating over-production of a protein the size of enolase 2 from S. cerevisiae cells transformed by the plasmid. One particular plasmid isolated from the Carlson gene bank that contained the EN02 gene was named pRD160.

### Isolation of EN02 Promoter

It was known from the literature that DNA sequences required for EN02 promoter activity are located between a SalI site at about -600 base pairs and the beginning of the EN02 coding sequence (Cohen et al. (1986) Mol. Cell Biol. 6: 2287). Therefore, the 750 base pair SalI to HindIII fragment that contained the EN02 promoter activity and the first few bases of ENO2 coding sequence from pRD160 was purified by acrylamide gel electrophoresis. This fragment was then partially digested with DdeI, and the 550 base pair SalI to DdeI (partial) fragment was purified from other fragments, again by acrylamide gel electrophoresis. This SalI to DdeI (partial) fragment, together with a synthetic double stranded DNA fragment, were used to reconstitute the EN02 promoter with a heterologous gene, the Aspergillus niger glucoamylase gene, as described below.

### Expression of a Heterologous Gene from the EN02 Promoter.

Referring to Fig. 1, the S. cerevisiae EN02 promoter was substituted for the TPI1 promoter in plasmid pRD158, as described below. (Abbreviations for restriction sites in the Figures are as follows: A, XbaI; C, ClaI; E, EcoRI; G, BglII; H, HindIII; L, BclI; M, XmaI; X, XhoI; S,SalI. Parentheses indicate a former site destroyed during construction. Moving clockwise around, pRD158 contains the following DNA fragments: (1) HindIII to XmaI, the S. cerevisiae URA3 gene; (2) XmaI, XhoI, XmaI, a synthetic adapter with the sequence 
5'-CCGGGCTCGAGC
      CGAGCTCGGGCC-5'
that created a XhoI site between two XmaI sites; (3) XmaI to BglII, the S. cerevisiae promoter, going from a naturally occurring MstII TPI1 site (filled in) at -638 to a T residue at -44, followed by a synthetic DNA fragment of the following sequence: 
5'-AGAATTCATCTATAACTACAAAAAACACATACATAAACTAAAAATGTCTTTCAGATCT-3'
3'-TCTTAAGTAGATATTGATGTTTTTTGTGTATGTATTTGATTTTTACAGAAAGTCTAGA-5'
(4) BglII to XbaI, a DNA copy of the A. niger glucoamylase gene (Boel et al. (1984) EMBO Journal 3: 1097). The BglII site was introduced artificially without changing the native amino acid sequence, and the XbaI site was inserted artificially at a natural BclI site (Boel et al., id.); (5) XbaI to HindIII, a 90 base pair fragment containing the TPI1 transcription terminator; (6) HindIII to (EcoRI), the majority of pBR322, with a 275 base pair SalI to BamHI deletion that leaves a SalI site but no BamHI site, and abolishes tetracycline resistance; (7) (EcoRI) to (EcoRI), an approximately 1000 base pair (XbaI) to (PstI) fragment containing the origin of replication from the endogenous S. cerevisiae 2 micron circle plasmid; (8) (EcoRI) to (ClaI), the short EcoRI to ClaI fragment of pBR322; (9) (ClaI) to (ClaI), an approximately 1200 base pair (ClaI) to (BamHI) fragment containing CEN3, the centromere from S. cerevisiae chromosome III; (10) (ClaI) to HindIII, the short ClaI to HindIII fragment of pBR322.

The XhoI to BglII fragment of pRD158 that contained the TPI1 promoter was substituted with the 550 base pair (SalI) to (DdeI) (partial) fragment described above, together with a synthetic 60/61-mer that contained sticky ends compatible with the DdeI and BglII sticky ends; the 60/61-mer has the sequence: 
5'-TTAGTTTCTTTCATAACACCAAGCAACTAATACTATAACATAC
3'-CAAAGAAAGTATTGTGGTTCGTTGATTATGATATTGTATG

AATAATAATGTCTTTCA-3'
TTATTATTACAGAAAGTCTAG-5'
The resulting plasmid, containing the S. cerevisiae EN02 promoter joined correctly to the glucoamylase gene via the synthetic fragment, was named pRD170. pRD170 was used to transform S. cerevisiae strain BWG 1-7A (Mata, leu 2-3, leu 2-112, his 4-519, ade 1-100, ura 3-52) (Guarente and Hoar (1984) Proc. Natl. Acad. Sci. USA 81: 7860) to URA3+. Transformants were grown to saturation in minimal selective medium containing 2% glucose (until all glucose had been consumed) and culture supernatants were assayed by incubating a mixture of 0.5 ml supernatant, 0.1 ml 1.0 M sodium citrate, pH 5.0, 0.2 ml 5% Difco soluble starch, and 0.3 ml water, at 50°C for 0.2 to 12 hours. At appropriate times, a 25 microliter aliquot from each sample was assayed for released glucose with a Yellow Springs Instruments Glucose Analyzer according to the manufacturer. The pRD170-transformed cells exhibited high glucoamylase production as measured by this test.

### Promoter Elements that Affect Heterologous Gene Expression

In S. cerevisiae, the best studied yeast, it is generally the case that a DNA fragment containing sequences from about -800 to +1 (relative to the A of the ATG of the start codon of the associated gene) will provide transcription and translation initiation, as well as mRNA stabilization, when the fragment is connected by ligation to a heterologous coding sequence. Usually such a promoter fragment contains sequences that confer not only the ability to bring about gene expression, but also sequences (called regulatory sequences) that affect the regulation of expression for the associated gene.

In addition, sometimes regulatory sequences can be found downstream from +1, as far as about +230. In some well-studied yeast promoters, the regulatory sequence is separated from the actual point (or points) at which mRNA transcription begins ("initiation points") by about 100 to 400 base pairs. (This is in contrast to the situation in E. coli where regulatory sequences are usually in close proximity (within about 20 base pairs) to the initiation points.) In yeast, if a regulatory sequence is upstream from the initiation points, it is called a UAS (upstream activation sequence) (see Guarente et al. (1982) Proc. Natl. Acad. Sci. U.S.A. 79: 7410). The UAS is usually between about 15 base pairs and 120 base pairs in length, and as mentioned above, often lies between -800 and the transcription initiation points. Unlike the situation for E. coli, the exact spacing between the UAS and initiation points is not critical, since it is known that UAS's can be moved tens or even hundreds of base pairs relative to transcription initiation points in either direction without abolishing UAS activity. Other sequences that are thought to play a role in expression of genes in yeast are 1) the so called "TATA" or "TATAA" sequence (which is located at -180 to -176 in S. cerevisiae ENO2), 2) the sequence CACACA or CATACA or a closely related sequence that is found between the initiation points and +1 of many highly expressed yeast genes (-12 to -7 in S. cerevisiae ENO2), 3) the sequences at the initiation points (in many yeast genes, transcription initiates at A residues), and 4) the sequences surrounding the ATG start codon. Many highly expressed Saccharomyces genes have an A at -3 and a G at +4, and G is statistically under-represented in the region upstream from the ATG (-1 to about -20). In yeast, except for "TATA", there is no consensus sequence for promoters or ribosome binding sites as in E. coli. Moreover, the precise spacing between sequence elements does not seem to be critical, since the distance between "TATA" and initiation points can vary from about 25 to 100 base pairs. All of the above-mentioned promoter elements can influence transcription, translation, or mRNA stability. Because of this fact, one can obtain advantages of the ENO2 promoters of this invention by using portions of the above-described ENO2 sequence of pRD170 in combination with other promoter elements derived from other sources. That such hybrid promoters can advantageously be made and used has been proven in the case of other yeast promoters, as follows.

As measured with lacZ fusions (Yocum et al. (1984) Mol. Cell. Biol. 4: 1985), a fully induced GAL10 promoter is about twice as strong as a fully induced CYC1 promoter (1800 vs. 900 units). However a hybrid promoter that substituted the GAL10 UAS for the CYC1 UAS, but retained the other 3 elements ("TATA", initiation points, and ATG region) of the CYC1 promoter was, when fully induced, almost twice as strong as the GAL10 promoter (3400 units). From this data one can conclude that the fully induced GAL10 UAS is more effective than the fully induced CYC1 UAS, but that the other three elements of CYC1 are more effective than the analogous sequences from GAL10, at least in the particular context of expressing the heterologous lacZ fusion.

Thus the entire ENO2 promoter region from -598 to +1, cloned as above, and used to express a heterologous gene, contains several smaller elements that will be useful in conjunction with other promoter elements from other promoters. For example, the ENO2 UAS is known to be situated between a SalI site at -598 and about -456. There is a BclI site at -315 and a BstXI site at -405. Thus the ENO2 UAS (by itself a "promoter" according to the definition used herein) can be easily obtained on a SalI to BclI or BstXI fragment and used to make hybrid promoters. For example, the TPI1 promoter has SphI and MstII sites at -225 and -638, respectively, which surround the TPI1 UAS. The ENO2 promoter could be substituted for the TPI1 UAS by ligating the ENO2 UAS (after blunting the ends) into the SphI to MstII gap suggested above (after blunting the ends). Similarly, the S. diastaticus DEX1 gene (Yamashita et al. (1985) J. Bact. 161: 567) has a Sau3A site at -92 that could be ligated to the BclI site of the ENO2 promoter to give a hybrid ENO2-DEX1 promoter.

Conversely, after removal of the ENO2 UAS on an appropriate plasmid such as pRD170 by cutting with SmaI and BclI, a new UAS with desirable regulatory properties (for example, the S. cerevisiae GAL1,10 UAS) could be inserted (Guarente et al. (1982) Proc. Natl. Acad. Sci. 79: 7410) to give a hybrid promoter that utilizes the "TATA", initiation points, and ATG start codon region of the ENO2 promoter. Other appropriate UAS's are also useful in hybrid promoters; for example, the S. carlsbergensis GAL1,10 UAS (Citron et al., supra). It is also possible to construct a hybrid promoter that contains two or more ENO2 UAS's, or an ENO2 UAS plus a TPI1 UAS, and so on.

### Construction of the ENO2-GAL1,10 Hybrid Promoter

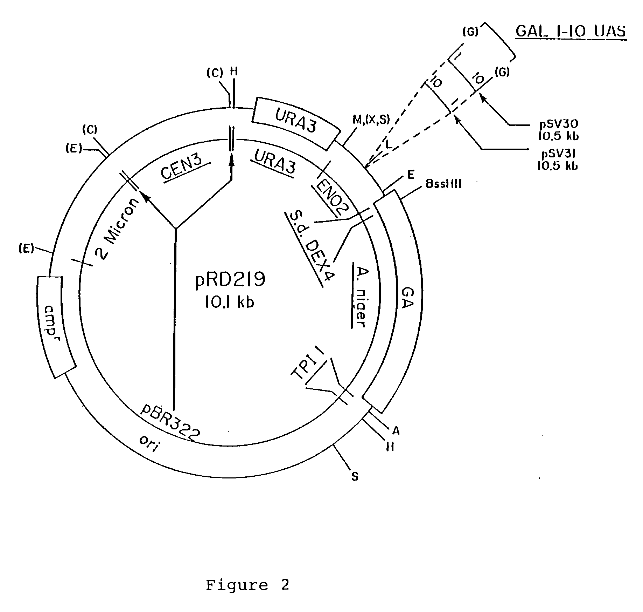

An ENO2-GAL1,10 hybrid promoter was constructed starting with the plasmid pRD219 (Fig. 2) which contains the ENO2 promoter fused to a glucoamylase gene. pRD219 is identical to pRD170 (above) with the exception of the junction region connecting the ENO2 promoter and the glucoamylase gene. The junction fragment consists of ENO2 promoter sequences derived from positions -50 to -1 (relative to translation initiation), followed by the DNA sequence coding for the S. diastaticus DEX4 gene signal peptide (described in Maine et al., U.S. Serial Number 810,423, filed December 18, 1985, assigned to the same assignee and hereby incorporated by reference). The presence of the DEX4 signal peptide encoding sequence allows for efficient secretion of the glucoamylase gene product. The junction fragment is a DdeI-BssHII 138/139-mer synthetic DNA fragment of the following sequence: 
5'-TTAGAATTCTTTCATAACACCAAGCAACTAATACT
3'-CTTAAGAAAGTATTGTGGTTCGTTGATTATGA

ATAACATACAATAATAATGGCAAGACAAAAGATGTTTT
TATTGTATGTTATTATTACCGTTCTGTTTTCTACAAAA

ATAACAAATTACTCGGCATGCTCAGCGTAGGATT
TATTGTTTAATGAGCCGTACGAGTCGCATCCTAA

CGGGTTTGCTTGGGCGTTGGAAAACATTAAG-3'
GCCCAAACGAACCCGCAACCTTTTGTAATTCGCGC-5'
The ENO2-derived region of the synthetic DNA fragment contains a single base change and a single base insertion relative to the natural ENO2 gene sequence just 3' to the DdeI site, that results in the introduction of an EcoRI site. The junction fragment joins the DEX4 signal peptide encoding DNA to the A. niger glucoamylase gene (Boel, et al., id.) through a BssHII site.

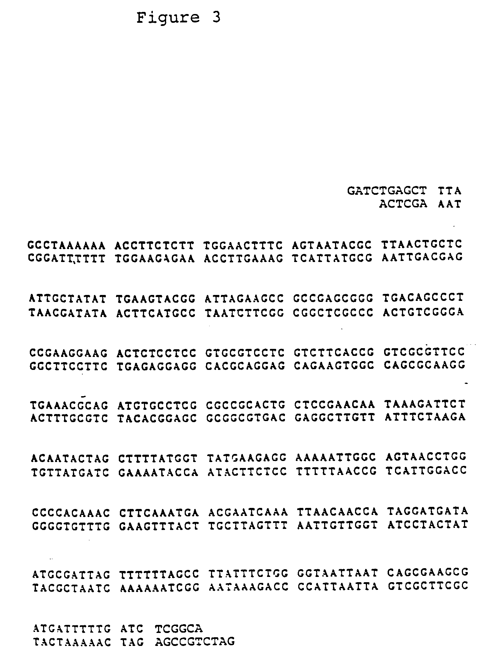

The following strategy was used to insert the S. cerevisiae GAL1,10 UAS between the ENO2 UAS and the ENO2 TATA region of pRD219. The 365 bp DdeI-Sau3A fragment which contains the GAL1,10 UAS (See Fig. 3) (Guarente et al. (1982) Proc. Natl. Acad. Sci. USA 79: 7410; Yocum et al. (1984) Mol. Cell. Biol.4: 1985) was purified, its ends were made flush by filling in, and it was ligated to the large HindIII-AvaI fragment of pBR322, which had also had its ends filled in, to form the plasmid pRY2O. In this construct, the HindIII and AvaI sites are regenerated. pRY20 was cut with AvaI, the ends were filled in, and it was ligated in the presence of 8 bp BglII linkers (New England Biolabs); the recircularized plasmid is pRY21. The same strategy was used to insert 8 bp BglII linkers at the HindIII site of pRY21, yielding pRY24. Finally, the approximately 375 bp BglII fragment of pRY24, containing the GAL1,10 UAS, was inserted in either orientation at the unique BclI site of pRD219, located at about position -316 relative to the translation initation codon of the DEX4-glucoamylase fusion gene, to form plasmids pSV30 and pSV31; pSV30 contains the GAL1,10 UAS oriented such that the GAL10 proximal end is closer to the glucoamylase gene, whereas pSV31 contains the GAL1,10 UAS in the opposite orientation.

pRD219, pSV30, and pSV31 were used to transform S. cerevisiae strain BWG 1-7A (MATa, leu2-3, leu2-112, his4-519, ade 1-100, ura3-52 GAL2) (Guarente and Hoar (1984) Proc. Natl. Acad. Sci., USA 81: 7860) to URA3+. Transformants were grown to saturation in minimal selective medium containing either 2% glucose or 2% galactose (until either all the glucose or all the galactose had been consumed), and the supernatants were assayed for glucoamylase activity as previously described. Glucoamylase activity was high in pRD219-transformed cells grown in glucose, but barely detectable in pSV30- and pSV31-transformed cells grown on glucose (see Table I), suggesting that the regulatory effects of the GAL1,10 UAS on glucoamylase expression are dominant over those of the ENO2 UAS. Glucose repression of the GAL1 and GAL10 genes is known to be mediated, at least in part, through sequences located in the GAL1,10 UAS (Yocum (1987) in Biological Research on Industrial Yeasts, Vol. III. eds. Stewart, Russell, Klein and Hiebsch (CRC Press, Boca Raton, Florida), pp. 61-70.).

Growth on galactose instead of glucose resulted in a reduction of glucoamylase production in cells containing pRD219, but an induction of glucoamylase production in cells containing pSV30 and pSV31, further supporting the above conclusion that the GAL1,10 UAS is dominant over the ENO2 UAS, in this context. The orientation of the GAL1,10 UAS had an effect on the level of galactose-induced expression of the glucoamylase gene; pSV30-transformed cells reproducibly exhibited higher levels of glucoamylase activity when grown on galactose than pSV31-transformed cells. Glucoamylase activity was 3 and 2 fold higher, respectively, in pSV30- and pSV31-transformed cells grown on galactose than in pRD219-transformed cells grown on glucose. Thus, the use of a hybrid promoter containing two UAS sequences resulted in a significant improvement in glucoamylase production, as well as strict regulation of glucoamylase production in response to a carbon source.

The effect of copy number of the ENO2-GAL1,10-glucoamylase fusion gene on glucoamylase production and carbon source regulation was also determined. The plasmids pRD219, pSV30 and pSV31 contain a centromere and are therefore maintained at about one copy per haploid genome. pRD166 is a precursor of pRD170 which lacks centromeric sequences, and pSV46 and pSV47 are derivatives of pSV30 and pSV31, respectively, which also lack centromeric sequences; these plasmids contain a 2-micron circle origin of replication and are therefore present in high copy number in the cell. pRD226, pSV52 and pSV53 are integrating vectors used to introduce single copies of the glucoamylase gene into the yeast genome; these plasmids are derived from pRD170, pSV30 and pSV31, respectively. A cir^{o} version of S. cerevisiae strain BWG 1-7A was used as a host yeast strain for experiments employing centromere plasmids and a cir⁺ version of this host strain was used for all other experiments.

Transformed cells were grown on either glucose or galactose and glucoamylase production was quantitated. Table 1 shows that when the hybrid ENO2-GAL1,10 promoter, containing two UAS sequences, directs expression of the glucoamylase gene, there is a significant increase in glucoamylase production, regardless of the copy number of the glucoamylase gene. pRD219, pRD166, and pRD266 do not contain the GAL1,10 UAS and produce approximately 2-3 fold less glucoamylase than the analogous GAL1,10 UAS-containing plasmids when fully induced. YEP24 does not contain a glucoamylase gene and, as expected, did not produce any glucoamylase. In addition, glucoamylase production in plasmids containing the GAL1,10 UAS, regardless of glucoamylase gene copy number, was induced by galactose, whereas plasmids lacking the GAL1,10 UAS , i.e. containing only the ENO2 UAS, were induced most efficiently, but not only, by glucose.

**Table 1**

| Plasmid | Type | Orientation of GAL1,10 UAS | Glucoamylase Activity* | |
|---|---|---|---|---|
| | | | Glucose | Galactose |
| pRD219 | centromere | ---- | 121 | 58 |
| pSV30 | centromere | GAL10 | 0 | 370 |
| pSV31 | centromere | GAL1 | 1 | 241 |
| pRD166 | 2-micron | ---- | 254 | 150 |
| pSV46 | 2-micron | GAL10 | 2 | 493 |
| pSV47 | 2-micron | GAL1 | 4 | 299 |
| pRD226 | integrating | ---- | 53 | 21 |
| pSV52 | integrating | GAL10 | 0 | 234 |
| pSV53 | integrating | GAL1 | 1 | 137 |
| YEP24 | 2-micron | ---- | 1 | 0 |

| | | | | |
|---|---|---|---|---|
| *arbitrary units | | | | |

### Construction of an ENO2-GAL1,10-PDGF fusion

The galactose inducible hybrid promoters containing ENO2 and GAL1,10 UAS sequences have also been used to express and secrete the human platelet-derived growth factor (PDGF) from yeast.

The ENO2 promoter for pRD219 or the hybrid promoters of pSV30 and pSV31 were placed in front of a hybrid gene encoding the DEX4 signal sequence fused in frame to PDGF coding sequences on autonomously replicating 2-micron based plasmids containing the selectable URA3 gene, to give pAP275A, pSV43, and pSV44, respectively. All three plasmids were transformed into a ura3⁻ haploid yeast by standard methods and the transformants were innoculated into 10 ml of minimal selective medium lacking uracil and containing either 2% glucose or 0.5% glucose plus 5% galactose as a carbon source. After 72 hours of growth at 30°C, the cells were removed and the culture supernatants were assayed for PDGF by Western blot using commercial anti-PDGF antibody (Collaborative Research, Waltham, MA). The level of secreted PDGF was estimated by comparison with a sample of PDGF isolated from human blood (Collaborative Research). As shown in Table 2, higher levels of PDGF were produced when the hybrid promoter directs expression of the PDGF gene than when the ENO2 promoter directs PDGF gene expression. The highest level of PDGF gene expression occurs when the GAL1 proximal end of the GAL1,10 UAS is oriented toward the PDGF gene (pSV44).

The presence of the plasmid pAP275A, which contains the PDGF gene under control of the ENO2 promoter, proved deleterious to growth of the transformed cell when glucose was the carbon source, presumably as a result of high level constitutive ENO2-promoted PDGF gene expression in the presence of the ENO2-inducer (i.e., glucose). Use of the regulated hybrid promoters (pSV43, pSV44) and growth of transformants in a mixture of glucose and galactose alleviated this problem. In the latter case, the hybrid promoter is glucose repressed in the early stages of fermentation and transformants grow at a normal rate and produce little or no PDGF; as the glucose is depleted later in fermentation, the hybrid promoter is derepressed by the lack of glucose and induced by galactose, leading to high levels of PDGF. Thus, the use of hybrid promoters containing strictly regulated promoters is clearly advantageous when expressing at high level a gene that hinders cell growth.

**Table 2**

| Plasmid | Carbon Source | Growth Rate | Secreted PDGF |
|---|---|---|---|
| pAP275A | 2%glucose | poor | 120 ng/ml |
| pSV43 | 0.5%glucose/5%galactose | good | 800 ng/ml |
| pSV44 | 0.5%glucose/5%galactose | good | 1200 ng/ml |

Hybrid promoters containing multiple UAS sequences may be useful for other applications. For instance, the glucoamylase gene on pRD219 could be made maltose-inducible by inserting the maltose UAS (UAS_{M}) at the BclI site. The UAS_{M} mediates induction of the maltase (MALS) and maltose permease (MALT) genes of S. cerevisiae by maltose (Hong and Marmur (1987) Mol Cell. Biol. 1: 247). A maltose-inducible glucoamylase gene could then be introduced into brewing strains of Saccharomyces to produce low calorie beer, or into distillery strains of Saccharomyces to increase carbohydrate utilization and ethanol yield.

It is possible to further subdivide the ENO2 promoter into individual functional sequences such as "TATA", initiation points, and ATG start codon region. Prepared as either restriction enzyme produced fragments or as synthetic oligonucleotides, these sequences could be substituted for the analogous sequence in other promoters, and such hybrid promoters may have desirable properties such as increased strength.

### Deposit

E. coli K12, strain YMC9, transformed with pRD170 was deposited in the American Type Culture Collection on April 10, 1987, and was assigned the accession number 67385.

## Claims

1. A DNA sequence comprising a yeast ENO2 promoter functionally fused to a heterologous gene, said gene not encoding an enolase.

2. A yeast cell transformed with the DNA sequence of claim 1.

3. The DNA sequence of claim 1 wherein said ENO2 promoter is derived from S. cerevisiae.

4. The yeast cell of claim 2, said yeast being of the genus Saccharomyces.

5. The DNA sequence of claim 1, contained in a plasmid vector capable of transforming a host yeast cell.

6. The DNA sequence of claim 1 wherein said heterologous gene encodes a glucoamylase.

7. Plasmid vector pRD170, ATCC No. 67385.

8. A method of producing a protein other than an enolase comprising growing a yeast cell transformed with a vector comprising a gene encoding said protein, said gene being functionally fused to an ENO2 promoter.

9. A DNA sequence comprising a hybrid yeast promoter functionally fused to a heterologous gene, said gene not encoding an enolase, said hybrid promoter comprising a DNA sequence comprising ENO2 promoter DNA functionally fused to a DNA sequence comprising a first upstream activating sequence derived from a yeast promoter.

10. The DNA sequence of claim 9 wherein said ENO2 promoter DNA includes an ENO2 upstream activating sequence.

11. The DNA sequence of claim 10 wherein said first upstream activating sequence is derived from a yeast promoter other than said ENO2 yeast promoter.

12. The DNA sequence of claim 10 wherein said first upstream activating sequence is derived from an ENO2 promoter.

13. The DNA sequence of claim 10 wherein said ENO2 promoter DNA includes a TATA region.

14. The DNA sequence of claim 13 wherein said first upstream activating sequence is located between said ENO2 upstream activating sequence and said TATA region of said ENO2 promoter.

15. The DNA sequence of claim 10, said first upstream activating sequence being a GAL1,10 upstream activating sequence having a GAL1-proximal end and a GAL10-proximal end.

16. The DNA sequence of claim 15, said GAL1-proximal end being closest to said heterologous gene.

17. The DNA sequence of claim 15, said GAL10-proximal end being closest to said heterologous gene.

18. The DNA sequence of claim 15, said GAL1,10 upstream activating sequence being derived from the GAL1,10 DNA sequence of yeast of the genus Saccharomyces.

19. The DNA sequence of claim 18, said yeast being S. cerevisiae.

20. A yeast cell transformed with the DNA sequence of claim 10.

21. The yeast cell of claim 20, said yeast being of the genus Saccharomyces.

22. A plasmid vector comprising the DNA sequence of claim 10, said vector being capable of transforming a host yeast cell.

23. The DNA sequence of claim 10 wherein said heterologous gene encodes a glucoamylase.

24. The vector pRD219, said vector comprising the vector pRD170, ATCC No: 67385, wherein the S. diastaticus DEX4 signal peptide encoding sequence is inserted 3' to the -1 position of the ENO2 promoter, said insertion comprising a DdeI-BssHII 138/139 - mer synthetic DNA fragment having the sequence

5'-TTAGAATTCTTTCATAACACCAAGCAACTAATACT
3'-CTTAAGAAAGTATTGTGGTTCGTTGATTATGA
ATAACATACAATAATAATGGCAAGACAAAAGATGTTTT
TATTGTATGTTATTATTACCGTTCTGTTTTCTACAAAA
ATAACAAATTACTCGGCATGCTCAGCGTAGGATT
TATTGTTTAATGAGCCGTACGAGTCGCATCCTAA
CGGGTTTGCTTGGGCGTTGGAAAACATTAAG-3'
GCCCAAACGAACCCGCAACCTTTTGTAATTCGCGC-5'

25. The vector pSV30, said vector comprising the vector pRD219, of Claim 24, wherein the GAL1, 10 UAS is inserted in the unique Bc1I site of pRD219, said GAL1,10 oriented such that the GAL10 proximal end is closer to the glucoamylase gene.

26. The vector, pSV31, said vector comprising the vector pRD219, of Claim 24, wherein the GAL1, 10 UAS is inserted in the unique Bc1I site of pRD219, said GAL1,10 was oriented such that the GAL10 proximal end is closer to the ENO2 promoter.

27. A method of producing a protein other than enolase 2 comprising growing a yeast cell transformed with a vector of claim 10.

## Patentansprüche

1. Eine DNA-Sequenz umfassend einen Hefe-ENO2-Promotor funktionell verbunden mit einem heterologen Gen, wobei das Gen nicht eine Enolase kodiert.

2. Eine Hefezelle, transformiert mit der DNA-Sequenz nach Anspruch 1.

3. Die DNA-Sequenz nach Anspruch 1, worin der ENO2-Promotor von S. cerevisiae stammt.

4. Die Hefezelle nach Anspruch 2, wobei die Hefe von der Gattung Saccharomyces ist.

5. Die DNA-Sequenz nach Anspruch 1, enthalten in einem Plasmidvektor, der fähig ist, eine Wirthefezelle zu transformieren.

6. Die DNA-Sequenz nach Anspruch 1, worin das heterologe Gen eine Glucoamylase kodiert.

7. Plasmidvektor pRD170, ATCC Nr. 67385.

8. Ein Verfahren zur Herstellung eines Proteins, das ein anderes als ein Enolase ist, umfassend die Zucht einer Hefezelle, die mit einem Vektor, der ein Gen umfaßt, das besagtes Protein kodiert, transformiert wurde, wobei das Gen funktionell mit einem ENO2-Promotor verbunden ist.

9. Eine DNA-Sequenz umfassend einen Hybridhefepromotor, der funktionell mit einem heterologen Gen verbunden ist, wobei das Gen keine Enolase kodiert, wobei der Hybridpromotor eine DNA-Sequenz umfaßt, die eine ENO2-Promotor-DNA umfaßt, die funktionell mit einer DNA-Sequenz verbunden ist, die eine erste stromaufwärts aktivierende Sequenz umfaßt, die von einem Hefepromotor abgeleitet ist.

10. Die DNA-Sequenz nach Anspruch 9, worin die ENO2-Promotor-DNA eine ENO2 stromaufwärts aktivierende Sequenz enthält.

11. Die DNA-Sequenz nach Anspruch 10, worin die erste stromaufwärts aktivierende Sequenz von einem Hefepromotor stammt, der ein anderer als der ENO2-Hefepromotor ist.

12. Die DNA-Sequenz nach Anspruch 10, worin die erste, stromaufwärts aktivierende Sequenz von einem ENO2-Promotor abgeleitet ist.

13. Die DNA-Sequenz nach Anspruch 10, worin die ENO2-Promotor-DNA eine TATA-Region enthält.

14. Die DNA-Sequenz nach Anspruch 13, worin sich die erste stromaufwärts aktivierende Sequenz zwischen der ENO2 stromaufwärts aktivierenden Sequenz und der TATA-Region des ENO2-Promotors befindet.

15. Die DNA-Sequenz nach Anspruch 10, wobei die erste stromaufwärts aktivierende Sequenz eine GAL1,10 stromaufwärts aktivierende Sequenz ist, die ein GAL1-nahes Ende und ein GAL10-nahes Ende hat.

16. Die DNA-Sequenz nach Anspruch 15, wobei sich das GAL1-nahe Ende am nähesten zum heterologen Gen befindet.

17. Die DNA-Sequenz nach Anspruch 15, wobei sich das GAL10-nahe Ende am nähesten zu dem heterologen Gen befindet.

18. Die DNA-Sequenz nach Anspruch 15, wobei die GAL1,10 stromaufwärts aktivierende Sequenz von der GAL1,10-DNA-Sequenz einer Hefe der Gattung Saccharomyces abgeleitet ist.

19. Die DNA-Sequenz nach Anspruch 18, wobei die Hefe S. cerevisiae ist.

20. Eine Hefezelle, die mit der DNA-Sequenz nach Anspruch 10 transformiert wurde.

21. Die Hefezelle nach Anspruch 20, wobei die Hefe von der Gattung Saccharomyces ist.

22. Ein Plasmidvektor umfassend die DNA-Sequenz nach Anspruch 10, wobei der Vektor in der Lage ist, eine Wirtshefezelle zu transformieren.

23. Die DNA-Sequenz nach Anspruch 10, worin das heterologe Gen eine Glucoamylase kodiert.

24. Der Vektor pRD219, wobei der Vektor den Vektor pRD170, ATCC Nr. 67385 umfaßt, worin die S. diastaticus DEX4-Signalpeptid kodierende Sequenz in 3' bei der -1-Position des ENO2-Promotors eingefügt ist, wobei der Einschub ein DdeI-BssHII 138/139-mer synthetisches DNA-Fragment mit der Sequenz 
5'-TTAGAATTCTTTCATAACACCAAGCAACTAATACT
3'-CTTAAGAAAGTATTGTGGTTCGTTGATTATGA
ATAACATACAATAATAATGGCAAGACAAAAGATGTTTT
TATTGTATGTTATTATTACCGTTCTGTTTTCTACAAAA
ATAACAAATTACTCGGCATGCTCAGCGTAGGATT
TATTGTTTAATGAGCCGTACGAGTCGCATCCTAA
CGGGTTTGCTTGGGCGTTGGAAAACATTAAG-3'
GCCCAAACGAACCCGCAACCTTTTGTAATTCGCGC-5'
 umfaßt.

25. Der Vektor pSV30, wobei der Vektor den Vektor pRD219 nach Anspruch 24, umfaßt, worin die GAL1,10 UAS in die einzige BclI-Schnittstelle von pRD219 eingefügt ist, wobei GAL1,10 so orientiert ist, daß das GAL10-nahe Ende näher an dem Glucoamylase-Gen ist.

26. Der Vektor pSV31, wobei der Vektor den Vektor pRD219 nach Anspruch 24 umfaßt, worin die GAL1,10 UAS in die einzige BclI-Schnittstelle von pRD219 eingefügt ist, wobei GAL1,10 so orientiert ist, daß das GAL10-nahe Ende näher an dem ENO2-Promotor ist.

27. Ein Verfahren zur Herstellung eines Proteins, das ein anderes als Enolase 2 ist, umfassend die Züchtung einer Hefezelle, die mit einem Vektor nach Anspruch 10 transformiert ist.

## Revendications

1. Séquence d'ADN comprenant un promoteur ENO2 de levure fusionné de manière fonctionnelle à un gène hétérologue, ledit gène ne codant pas une énolase.

2. Cellule de levure transformée avec la séquence d'ADN selon la revendication 1.

3. Séquence d'ADN selon la revendication 1, dans laquelle ledit promoteur ENO2 est dérivé de S. cerevisiae.

4. Cellule de levure selon la revendication 2, ladite levure étant du genre Saccharomyces.

5. Séquence d'ADN selon la revendication 1, contenue dans un vecteur plasmidique capable de transformer une cellule de levure hôte.

6. Séquence d'ADN selon la revendication 1, dans laquelle ledit gène hétérologue code une glucoamylase.

7. Vecteur plasmidique pRD170, ATCC n° 67385.

8. Procédé de production d'une protéine autre qu'une énolase, comprenant la culture d'une cellule de levure transformée avec un vecteur comprenant un gène codant ladite protéine, ledit gène étant fusionné de manière fonctionnelle à un promoteur ENO2.

9. Séquence d'ADN comprenant un promoteur de levure hybride fusionné de manière fonctionnelle à un gène hétérologue, ledit gène ne codant pas une énolase, ledit promoteur hybride comprenant une séquence d'ADN comprenant un ADN de promoteur ENO2 fusionné de manière fonctionnelle à une séquence d'ADN comprenant une première séquence activatrice en amont dérivée d'un promoteur de levure.

10. Séquence d'ADN selon la revendication 9, dans laquelle ledit ADN de promoteur ENO2 comprend une séquence activatrice en amont ENO2.

11. Séquence d'ADN selon la revendication 10, dans laquelle ladite première séquence activatrice en amont est dérivée d'un promoteur de levure autre que ledit promoteur de levure ENO2.

12. Séquence d'ADN selon la revendication 10, dans laquelle ladite première séquence activatrice en amont est dérivée d'un promoteur ENO2.

13. Séquence d'ADN selon la revendication 10, dans laquelle ledit ADN de promoteur ENO2 comprend une région TATA.

14. Séquence d'ADN selon la revendication 13, dans laquelle ladite première séquence activatrice en amont est située entre ladite séquence activatrice en amont ENO2 et ladite région TATA dudit promoteur ENO2.

15. Séquence d'ADN selon la revendication 10, ladite première séquence activatrice en amont étant une séquence activatrice en amont GAL1,10 ayant une extrémité GAL1-proximale et une extrémité GAL10-proximale.

16. Séquence d'ADN selon la revendication 15, ladite extrémité GAL1-proximale étant la plus proche dudit gène hétérologue.

17. Séquence d'ADN selon la revendication 15, ladite extrémité GAL10-proximale étant la plus proche dudit gène hétérologue.

18. Séquence d'ADN selon la revendication 15, ladite séquence activatrice en amont GAL1,10 étant dérivée de la séquence d'ADN GAL1,10 de levure du genre Saccharomyces.

19. Séquence d'ADN selon la revendication 18, ladite levure étant S. cerevisiae.

20. Cellule de levure transformée avec la séquence d'ADN selon la revendication 10.

21. Cellule de levure selon la revendication 20, ladite levure étant du genre Saccharomyces.

22. Vecteur plasmidique comprenant la séquence d'ADN selon la revendication 10, ledit vecteur étant capable de transformer une cellule de levure hôte.

23. Séquence d'ADN selon la revendication 10, dans laquelle ledit gène hétérologue code une glucoamylase.

24. Vecteur pRD219, ledit vecteur comprenant le vecteur pRD170, ATCC n° 67385, dans lequel la séquence codant le peptide signal DEX4 de S. diastaticus est insérée en 3' de la position -1 du promoteur ENO2, ladite insertion comprenant un fragment d'ADN synthétique 138/139 - mère DdeI-BssHII ayant la séquence

5'-TTAGAATTCTTTCATAACACCAAGCAACTAATACT
3'-CTTAAGAAAGTATTGTGGTTCGTTGATTATGA
ATAACATACAATAATAATGGCAAGACAAAAGATGTTTT
TATTGTATGTTATTATTACCGTTCTGTTTTCTACAAAA
ATAACAAATTACTCGGCATGCTCAGCGTAGGATT
TATTGTTTAATGAGCCGTACGAGTCGCATCCTAA
CGGGTTTGCTTGGGCGTTGGAAAACATTAAG-3'
GCCCAAACGAACCCGCAACCTTTTGTAATTCGCGC-5'

25. Vecteur pSV30, ledit vecteur comprenant le vecteur pRD219 selon la revendication 24, dans lequel la séquence activatrice en amont GAL1,10 est insérée dans l'unique site BclI de pRD219, ladite séquence activatrice en amont GAL1,10 étant orientée de manière que l'extrémité GAL10-proximale soit plus proche du gène de glucoamylase.

26. Vecteur pSV31, ledit vecteur comprenant le vecteur pRD219 selon la revendication 24, dans lequel la séquence activatrice en amont GAL1,10 est insérée dans l'unique site BclI de pRD219, ladite séquence activatrice en amont GAL1,10 étant orientée de manière que l'extrémité GAL10-proximale soit plus proche du promoteur ENO2.

27. Procédé de production d'une protéine autre que l'énolase 2, comprenant la culture d'une cellule de levure transformée avec un vecteur selon la revendication 10.
